(19) Europäisches Patentamt
European Patent Office
Office européen des brevets

(11) Veröffentlichungsnummer: **0 258 785**
**A2**

# (12) EUROPÄISCHE PATENTANMELDUNG

(21) Anmeldenummer: **87112307.1**

(22) Anmeldetag: **25.08.87**

(51) Int. Cl.⁴: **C07D 249/08**, A01N 43/653, C07D 405/06

(30) Priorität: **04.09.86 DE 3630129**

(43) Veröffentlichungstag der Anmeldung: **09.03.88 Patentblatt 88/10**

(84) Benannte Vertragsstaaten: **AT BE CH DE FR GB IT LI NL SE**

(71) Anmelder: **BAYER AG**
**Konzernverwaltung RP Patentabteilung**
**D-5090 Leverkusen 1 Bayerwerk(DE)**

(72) Erfinder: **Büchel, Karl-Heinz, Prof. Dr.**
**Dabringhausener Strasse 42**
**D-5093 Burscheid(DE)**
Erfinder: **Holmwood, Graham, Dr.**
**Krutscheider Weg 105**
**D-5600 Wuppertal(DE)**
Erfinder: **Kraatz, Udo, Dr.**
**Andreasstrasse 22a**
**D-5090 Leverkusen 1(DE)**
Erfinder: **Krämer, Wolfgang, Dr.**
**Rosenkranz 25**
**D-5093 Burscheid 2(DE)**
Erfinder: **Reinecke, Paul, Dr.**
**Steinstrasse 8**
**D-5090 Leverkusen 3(DE)**
Erfinder: **Brandes, Wilhelm, Dr.**
**Eichendorffstrasse 3**
**D-5653 Leichlingen(DE)**
Erfinder: **Stendel, Wilhelm, Dr.**
**In den Birken 55**
**D-5600 Wuppertal 1(DE)**

(54) **Mercapto-substituierte Hydroxyethyl-(triazol-1-yl)-Derivate.**

(57) Neue mercapto-substituierte Hydroxyethyl-(triazol-1-yl)-Derivate der Formel

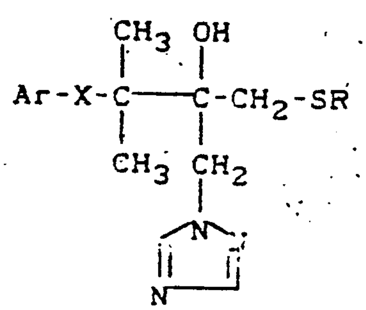

(I)

in welcher
Ar für gegebenenfalls substituiertes Aryl steht,

EP 0 258 785 A2

X für Sauerstoff, Schwefel, eine direkte Bindung sowie für die Gruppierungen $-CH_2-$, $-CH_2-CH_2$, $-O-CH_2-$, $-SCH_2-$, $-O-CH_2-CH_2-$und $-S-CH_2-CH_2-$steht, wobei das Heteroatom mit dem Aryl-Rest verbunden ist, wenn X für $-OCH_2-$, $-SCH_2-$, $-O-CH_2-CH_2-$oder $-S-CH_2-CH_2-$steht, und

R für Wasserstoff, gegebenenfalls substituiertes Alkyl, gegebenenfalls substituiertes Alkenyl, gegebenenfalls substituiertes Alkinyl, gegebenenfalls substituiertes Cycloalkyl, gegebenenfalls substituiertes Cycloalkylalkyl, gegebenenfalls substituiertes Aralkyl oder für gegebenenfalls substituiertes Phenyl steht,

sowie deren Säureadditions-Salze und Metallsalz-Komplexe, ein Verfahren zur Herstellung der neuen Stoffe und deren Verwendung als Fungizide und Parasitizide.

Neue Oxirane der Formel

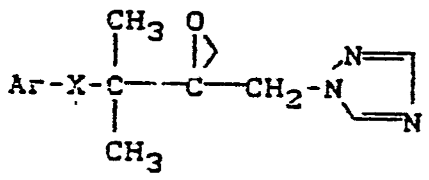

(II)

in welcher

Ar und X die oben angegebene Bedeutung haben,

sowie Verfahren zur Herstellung dieser Oxirane, die als Zwischenprodukte zur Herstellung von Stoffen der Formel (I) dienen.

## Mercapto-substituierte Hydroxyethyl-(triazol-1-yl)-Derivate

Die vorliegende Erfindung betrifft neue mercapto-substituierte Hydroxyethyl-(triazol-1-yl)-Derivate, ein Verfahren zu ihrer Herstellung und ihre Verwendung als Fungizide und Parasitizide.

Es ist bereits bekannt geworden, daß bestimmte 1-Hydroxyalkyl-triazolyl-Derivate fungizide Eigenschaften besitzen (vgl. EP-OS 0 061 835, EP-OS 0 084 834 und EP-OS 086 173). So können z.B. 4-Fluor-2-(4-chlor-phenyl-ethyl)-3,3-dimethyl-1-(1,2,4-triazol-1-yl)-butan-2-ol und 4-Fluor-2-(4-chlor-phenyl-thiomethyl)-3,3-dimethyl-1-(1,2,4-triazol-1-yl)-butan-2-ol zur Bekämpfung von Pilzen eingesetzt werden. Die Wirksamkeit dieser Stoffe ist jedoch vor allem bei niedrigen Aufwandmengen und Konzentrationen nicht immer ganz befriedigend. Außerdem ist ihre Verwendung als Parasitizide nicht bekannt.

Es wurden nun mercapto-substituierte Hydroxyethyl-(triazol-1-yl)-Derivate der Formel

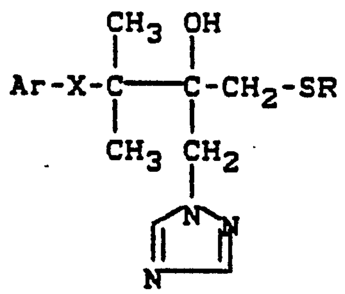

(I)

in welcher

Ar für gegebenenfalls substituiertes Aryl steht,

X für Sauerstoff, Schwefel, eine direkte Bindung sowie für die Gruppierungen $-CH_2-$, $-CH_2-CH_2-$, $-O-CH_2-$, $-SCH_2-$, $-O-CH_2-CH_2-$und $-S-CH_2-CH_2-$steht, wobei das Heteroatom mit dem Aryl-Rest verbunden ist, wenn X für $-OCH_2-$, $-SCH_2-$, $-O-CH_2-CH_2$ oder $-S-CH_2-CH_2-$steht, und

R für Wasserstoff, gegebenenfalls substituiertes Alkyl, gegebenenfalls substituiertes Alkenyl, gegebenenfalls substituiertes Alkinyl, gegebenenfalls substituiertes Cycloalkyl, gegebenenfalls substituiertes Cycloalkylalkyl, gegebenenfalls-substituiertes Aralkyl oder für gegebenenfalls substituiertes Phenyl steht,

sowie deren Säureadditions-Salze und Metallsalz-Komplexe gefunden.

Die neuen mercapto-substituierten Hydroxyethyl-(triazol-1-yl)-Derivate der Formel (I) besitzen ein asymmetrisch substituiertes Kohlenstoffatom und können deshalb in den beiden optischen Isomerenformen anfallen. Die Erfindung betrifft sowohl die Isomerengemische als auch die einzelnen Isomeren.

Weiterhin wurde gefunden, daß man mercapto-substituierte Hydroxyethyl-(triazol-1-yl)-Derivate der Formel (I) sowie deren Säureadditions-Salze und Metallsalz-Komplexe erhält, wenn man Oxirane der Formel

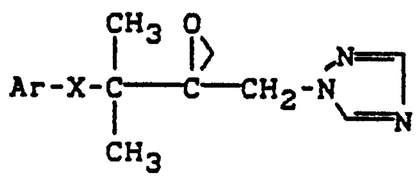

(II)

in welcher

Ar und X die oben angegebene Bedeutung haben,

mit Mercaptanen der Formel

R-SH (III)

in welcher

R die oben angegebene Bedeutung hat,

in Gegenwart eines Verdünnungsmittels und gegebenenfalls in Gegenwart eines Säurebindemittels umsetzt und gegebenenfalls anschließend an die so erhaltenen Verbindungen der Formel (I) eine Säure oder ein Metallsalz addiert.

Außerdem wurde gefunden, daß sich die neuen mercapto-substituierten Hydroxyethyl-(triazol-1-yl)-Derivate der Formel (I) sowie deren Säureadditions-Salze und Metallsalz-Komplexe durch sehr gute fungizide und parasitizide Eigenschaften auszeichnen.

Überraschenderweise besitzen die erfindungsgemäßen mercapto-substituierten Hydroxyethyl-(triazol-1-yl)-Derivate der Formel (I) sowie deren Säureadditions-Salze und Metallsalz-Komplexe eine bessere fungizide Wirkung als das 4-Fluor-2-(4-chlor-phenyl-ethyl)-3,3-dimethyl-1-(1,2,4-triazol-1-yl)-butan-2-ol und das 4-Fluor-2-(4-chlor-phenyl-thiomethyl)-3,3-dimethyl-1-(1,2,4-triazol-1-yl)-butan-2-ol, welches konstitutionell ähnliche, vorbekannte Wirkstoffe gleicher Wirkungsrichtung sind. Außerdem zeichnen sich die erfindungsgemäßen mercapto-substituierten Hydroxyethyl-(triazol-1-yl)-Derivate der Formel (I) sowie deren Säureadditions-Salze und Metallsalz-Komplexe auch durch eine parasitizide Wirkung aus.

Die erfindungsgemäßen mercapto-substituierten Hydroxyethyl-(triazol-1-yl)-Derivate sind durch die Formel (I) allgemein definiert. Bevorzugt sind diejenigen Verbindungen der Formel (I), in denen

Ar für Aryl mit 6 bis 10 Kohlenstoffatomen steht, welches einfach oder mehrfach, gleichartig oder verschieden substituiert sein kann durch Halogen, gegebenenfalls substituiertes Alkyl, gegebenenfalls substituiertes Alkoxy, gegebenenfalls substituiertes Alkylthio, gegebenenfalls substituiertes Alkenyl, gegebenenfalls substituiertes Alkinyl, gegebenenfalls substituiertes Phenyl, gegebenenfalls substituiertes Phenoxy, gegebenenfalls substituiertes Phenylalkyl, Nitro, Cyano, Formyl, Alkylcarbonyl und/oder von Formyl oder Alkylcarbonyl abgeleitete Oxime, Ketale und Hydrazone,

X für Sauerstoff, Schwefel, eine direkte Bindung sowie für die Gruppierungen $-CH_2-$, $-CH_2-CH_2-$, $-O-CH_2-$, $-SCH_2-$, $-O-CH_2-CH_2-$und $-S-CH_2-CH_2-$steht, wobei das Heteroatom mit dem Aryl-Rest verbunden ist, wenn X für $-OCH_2-$, $-SCH_2-$, $-O-CH_2-CH_2-$oder $-S-CH_2-CH_2-$steht, und

R für Wasserstoff, gegebenenfalls substituiertes, geradkettiges oder verzweigtes Alkyl mit 1 bis 12 Kohlenstoffatomen, gegebenenfalls substituiertes Alkenyl mit 2 bis 12 Kohlenstoffatomen, gegebenenfalls substituiertes Alkinyl mit 3 bis 8 Kohlenstoffatomen, gegebenenfalls einfach oder zweifach, gleichartig oder verschieden durch Halogen und/oder Alkyl substituiertes Cycloalkyl mit 3 bis 6 Kohlenstoffatomen, gegebenenfalls einfach oder zweifach, gleichartig oder verschieden durch Halogen und/oder Alkyl substituiertes Cycloalkylalkyl mit 3 bis 6 Kohlenstoffatomen im Cycloalkylteil und 1 oder 2 Kohlenstoffatomen im Alkylteil, für Aralkyl mit 6 bis 10 Kohlenstoffatomen im Arylteil und 1 bis 4 Kohlenstoffatomen im Alkylteil steht, wobei der Arylteil einfach oder mehrfach, gleichartig oder verschieden substituiert sein kann durch Halogen, gegebenenfalls substituiertes Alkyl, gegebenenfalls substituiertes Alkoxy, gegebenenfalls substituiertes Alkylthio, gegebenenfalls substituiertes Alkenyl, gegebenenfalls substituiertes Alkinyl, gegebenenfalls substituiertes Phenyl, gegebenenfalls substituiertes Phenoxy, gegebenenfalls substituiertes Phenylalkyl, Nitro, Cyano, Formyl, Alkylcarbonyl und/oder von Formyl oder Alkylcarbonyl abgeleitete Oxime, Ketale und Hydrazone, oder

R für gegebenenfalls durch Halogen und/oder Alkyl substituiertes Phenyl steht.

Eine besonders bevorzugte Gruppe erfindungsgemäßer Stoffe sind diejenigen mercapto-substituierten Hydroxyethyl-(triazol-1-yl)-Derivate der Formel (I), in denen

Ar für Phenyl steht, welches einfach bis dreifach, gleichartig oder verschieden substituiert sein kann durch Halogen, Alkyl mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen und/oder Phenyl,

X für Sauerstoff, Schwefel, eine direkte Bindung sowie für die Gruppierungen $-CH_2-$oder $-CH_2-CH_2-$steht, und

R für Wasserstoff, gegebenenfalls substituiertes, geradkettiges oder verzweigtes Alkyl mit 1 bis 12 Kohlenstoffatomen, gegebenenfalls substituiertes Alkenyl mit 2 bis 12 Kohlenstoffatomen, gegebenen falls substituiertes Alkinyl mit 3 bis 8 Kohlenstoffatomen, gegebenenfalls einfach oder zweifach, gleichartig oder verschieden durch Halogen und/oder Alkyl substituiertes Cycloalkyl mit 3 bis 6 Kohlenstoffatomen, gegebenenfalls einfach oder zweifach, gleichartig oder verschieden durch Halogen und/oder Alkyl substituiertes Cycloalkylalkyl mit 3 bis 6 Kohlenstoffatomen im Cycloalkylteil und 1 oder 2 Kohlenstoffatomen im Alkylteil oder für Aralkyl mit 6 bis 10 Kohlenstoffatomen im Arylteil und 1 bis 4 Kohlenstoffatomen im Alkylteil steht, wobei der Arylteil einfach oder mehrfach, gleichartig oder verschieden substituiert sein kann durch Halogen, gegebenenfalls substituiertes Alkyl, gegebenenfalls substituiertes Alkoxy, gegebenenfalls substituiertes Alkylthio, gegebenenfalls substituiertes Alkenyl, gegebenenfalls substituiertes Alkinyl, gegebenenfalls substituiertes Phenyl, gegebenenfalls substituiertes Phenoxy, gegebenenfalls substituiertes Phenylalkyl, Nitro, Cyano, Formyl, Alkylcarbonyl und/oder von Formyl oder Alkylcarbonyl abgeleitete Oxime, Ketale und Hydrazone, oder

R für ein-bis dreifach durch Halogen und/oder Alkyl mit 1 bis 4 Kohlenstoffatomen substituiertes Phenyl steht.

Eine weitere besonders bevorzugte Gruppe erfindungsgemäßer Stoffe sind diejenigen mercapto-substituierten Hydroxyethyl-(triazol-1-yl)-Derivate der Formel (I), in denen

Ar für Phenyl steht, welches einfach bis dreifach, gleichartig oder verschieden substituiert sein kann durch Halogen, Alkyl mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, Phenyl, Formyl und Acetyl und/oder von Formyl oder Acetyl abgeleitete Oximether,

X für die Gruppierungen $-O-CH_2-$, $-S-CH_2-$, $-O-CH_2-CH_2-$oder $-S-CH_2-CH_2-$steht, wobei das Heteroatom jeweils mit dem Phenyl-Rest verbunden ist, und

R für Wasserstoff, gegebenenfalls substituiertes, geradkettiges oder verzweigtes Alkyl mit 1 bis 12 Kohlenstoffatomen, gegebenenfalls substituiertes Alkenyl mit 2 bis 12 Kohlenstoffatomen, gegebenenfalls substituiertes Alkinyl mit 3 bis 8 Kohlenstoffatomen, gegebenenfalls einfach oder zweifach, gleichartig oder verschieden durch Halogen und/oder Alkyl substituiertes Cycloalkyl mit 3 bis 6 Kohlenstoffatomen, gegebenenfalls einfach oder zweifach, gleichartig oder verschieden durch Halogen und/oder Alkyl substituiertes Cycloalkylalkyl mit 3 bis 6 Kohlenstoffatomen im Cycloalkylteil und 1 oder 2 Kohlenstoffatomen im Alkylteil oder für Aralkyl mit 6 bis 10 Kohlenstoffatomen im Arylteil und 1 bis 4 Kohlenstoffatomen im Alkylteil steht, wobei der Arylteil einfach oder mehrfach, gleichartig oder verschieden substituiert sein kann durch Halogen, gegebenenfalls substituiertes Alkyl, gegebenenfalls substituiertes Alkoxy, gegebenenfalls substituier tes Alkylthio, gegebenenfalls substituiertes Alkenyl, gegebenenfalls substituiertes Alkinyl, gegebenenfalls substituiertes Phenyl, gegebenenfalls substituiertes Phenoxy, gegebenenfalls substituiertes Phenylalkyl, Nitro, Cyano, Formyl, Alkylcarbonyl und/oder von Formyl oder Alkylcarbonyl abgeleitete Oxime, Ketale und Hydrazone, oder

R für gegebenenfalls ein-bis dreifach durch Halogen und/oder Alkyl mit 1 bis 4 Kohlenstoffatomen substituiertes Phenyl steht.

Ganz besonders bevorzugt sind Verbindungen der Formel (I), in denen

Ar für Phenyl steht, welches einfach bis dreifach, gleichartig oder verschieden substituiert sein kann durch Fluor, Chlor, Brom, Methyl, Ethyl, Isopropyl, Butyl, Trifluormethyl, Trifluormethoxy, Tetrafluorethoxy, Trifluormethylmercapto, Tetrafluorethylmercapto und/oder Phenyl,

X für Sauerstoff sowie für die $-CH_2-$Gruppe steht und

R für Wasserstoff, gegebenenfalls durch Halogen substituiertes Alkyl mit 1 bis 6 Kohlenstoffatomen, gegebenenfalls durch Halogen substituiertes Alkenyl mit 2 bis 4 Kohlenstoffatomen, gegebenenfalls durch Halogen substituiertes Alkinyl mit 3 bis 6 Kohlenstoffatomen, gegebenenfalls ein-oder zweifach, gleichartig oder verschieden durch Fluor, Chlor und/oder Methyl substituiertes Cycloalkyl mit 3 bis 6 Kohlenstoffatomen, gegebenenfalls ein-oder zweifach, gleichartig oder verschieden durch Fluor, Chlor und/oder Methyl substituiertes Cycloalkylalkyl mit 3 bis 6 Kohlenstoffatomen im Cycloalkylteil und 1 oder 2 Kohlenstoffatomen im Alkylteil oder für Benzyl, welches im Phenylteil einfach bis dreifach, gleichartig oder verschieden substituiert sein kann durch Fluor, Chlor, Brom, Methyl, Ethyl, Isopropyl, Butyl, Trifluormethyl, Trifluormethoxy, Tetrafluorethoxy, Trifluormethylmercapto und/oder Tetrafluorethylmercapto, oder

R für gegebenenfalls ein-oder zweifach durch Fluor, Chlor und/oder Methyl substituiertes Phenyl steht.

Ganz besonders bevorzugt sind auch Verbindungen der Formel (I), in denen

Ar für Phenyl steht, welches einfach bis dreifach, gleichartig oder verschieden substituiert sein kann durch Fluor, Chlor, Brom, Methyl, Ethyl, Isopropyl, Butyl, Trifluormethyl, Trifluormethoxy, Tetrafluorethoxy, Trifluormethylmercapto, Tetrafluorethylmercapto, Formyl, Acetyl, Methoximinomethyl, Ethoximinomethyl, Methoximinoethyl, Ethoximinoethyl und/oder Phenyl,

X für die Gruppierungen $-O-CH_2-$, $-S-CH_2-$, $-O-CH_2-CH_2-$oder $-S-CH_2-CH_2$ steht, wobei das Heteroatom mit dem Phenylrest verbunden ist, und

R für Wasserstoff, gegebenenfalls durch Halogen sub stituiertes Alkyl mit 1 bis 6 Kohlenstoffatomen, gegebenenfalls durch Halogen substituiertes Alkenyl mit 2 bis 4 Kohlenstoffatomen, gegebenenfalls durch Halogen substituiertes Alkinyl mit 3 bis 6 Kohlenstoffatmen, gegebenenfalls ein-oder zweifach, gleichartig oder verschieden durch Fluor, Chlor und/oder Methyl substituiertes Cycloalkyl mit 3 bis 6 Kohlenstoffatomen, gegebenenfalls ein-oder zweifach, gleichartig oder verschieden durch Fluor, Chlor und/oder Methyl substituiertes Cycloalkylalkyl mit 3 bis 6 Kohlenstoffatomen im Cycloalkylteil und 1 oder 2 Kohlenstoffatomen im Alkylteil oder für Benzyl, welches im Phenylteil einfach bis dreifach, gleichartig oder verschieden substituiert sein kann durch Fluor, Chlor, Brom, Methyl, Ethyl, Isopropyl, Butyl, Trifluormethyl, Trifluormethoxy, Tetrafluorethoxy, Trifluormethylmercapto und/oder Tetrafluorethylmercapto, oder

R für gegebenenfalls ein-oder zweifach durch Fluor, Chlor und/oder Methyl substituiertes Phenyl steht.

Von ganz besonderem Interesse sind weiterhin solche Verbindungen der Formel (II), in denen

Ar für Phenyl steht, welches einfach oder zweifach, gleichartig oder verschieden substituiert sein kann durch Fluor, Chlor, Brom, Methyl, Ethyl, Isopropyl, Butyl, Trifluormethyl, Trifluormethoxy, Tetrafluorethoxy, Trifluormethylmercapto, Tetrafluorethylmercapto, Formyl, Acetyl, Methoximinomethyl, Ethox iminomethyl, Methoximinoethyl, Ethoximinoethyl und/oder Phenyl,

X für die Gruppierungen $-O-CH_2-$oder $-OCH_2-CH_2-$steht, wobei das Sauerstoffatom mit dem Phenylrest verbunden ist und

R für Wasserstoff, Methyl, Ethyl, Isopropyl, n-Propyl, n-Butyl, iso-Butyl, tert.-Butyl, n-Hexyl, Allyl, Propinyl, Cyclohexyl, Dichlorcyclopropyl, Dichlorcyclopropyl-methyl, Methylcyclohexyl, 4-Chlorbenzyl oder 4-Chlorphenyl steht.

Von ganz besonderem Interesse sind schließlich auch solche Verbindungen der Formel (I), in denen

Ar für Phenyl steht, welches einfach oder zweifach gleichartig oder verschieden substituiert sein kann durch Fluor, Chlor, Brom, Methyl, Ethyl, Isopropyl, Butyl, Trifluormethyl, Trifluormethoxy, Tetrafluorethoxy, Trifluormethylmercapto, Tetrafluorethylmercapto und/oder Phenyl,

X für Sauerstoff oder die Gruppierung $-CH_2-$steht und

R für Wasserstoff, Methyl, Ethyl, Isopropyl, n-Propyl, n-Butyl, iso-Butyl, tert.-Butyl, n-Hexyl, Allyl, Propinyl, Cyclohexyl, Dichlorcyclopropyl, Dichlorcyclopropylmethyl, Methylcyclohexyl, 4-Chlorbenzyl oder 4-Chlorphenyl steht.

Bevorzugte erfindungsgemäße Verbindungen sind auch Additionsprodukte aus Säuren und denjenigen mercapto-substituierten Hydroxyethyl-(triazol-1-yl)-Derivaten der Formel (I), in denen Ar, X und R diejenigen Bedeutungen haben, die im Zusammenhang mit der Beschreibung der erfindungsgemäßen Stoffe bereits vorzugsweise für diese Substituenten genannt wurden.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, mono-und bifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure und Milchsäure, sowie Sulfonsäuren, wie z.B. p-Toluolsulfonsäure und 1,5-Naphthalindisulfonsäure.

Außerdem bevorzugte erfindungsgemäße Verbindungen sind Additionsprodukte aus Salzen von Metallen der II. bis IV. Haupt-und der I. und II. sowie IV. bis VIII. Nebengruppe des Periodensystems der Elemente und denjenigen mercapto-substituierten Hydroxyethyl-(triazol-1-yl)-Derivaten der Formel (I), in denen Ar, X und R die Bedeutungen haben, die bereits vorzugsweise für diese Substituenten genannt wurden.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Verwendet man beispielsweise 2-(4-Chlorphenoxy-tert.-butyl)-2-(1,2,4-triazol-1-yl-methyl)-oxiran und Methylmercaptan als Ausgangssubstanzen, so kann der Verlauf des erfindungsgemäßen Verfahrens durch das folgende Formelschema wiedergegeben werden:

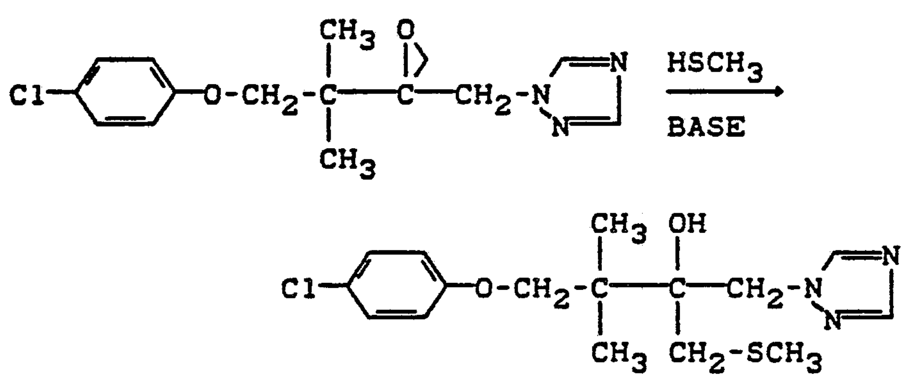


Die bei dem erfindungsgemäßen Verfahren als Ausgangsstoffe benötigten Oxirane sind durch die Formel (II) allgemein definiert. In dieser Formel haben Ar und X bevorzugt diejenigen Bedeutungen, die bereits im Zusammenhang mit der Beschreibung der erfindungsgemäßen Stoffe der Formel (I) bevorzugt für diese Reste genannt wurden.

Die Oxirane der Formel (II) sind neu. Sie lassen sich herstellen, indem man Ketone der Formel

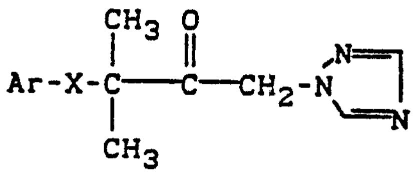
(IV)

in welcher
Ar und X die oben angegebene Bedeutung haben,
entweder
α) mit Dimethyloxosulfonium-methylid der Formel

$$\overset{\delta^{+}\delta^{-}}{(CH_3)_2SOCH_2} \qquad (V)$$

in Gegenwart eines Verdünnungsmittels, wie beispielsweise Dimethylsulfoxid, bei Temperaturen zwischen 20°C und 80°C umsetzt,
oder
β) mit Trimethylsulfonium-methylsulfat der Formel
$[(CH_3)_3S^{\oplus}]\ CH_3SO_4^{\ominus}$ (VI)
in Gegenwart eines inerten organischen Lösungsmittels, wie z.B. Acetonitril, und in Gegenwart einer Base, wie z.B. Natriummethylat, bei Temperaturen zwischen 0°C bis 60°C, vorzugsweise bei Raumtemperatur, umsetzt.

Die Oxirane der Formel (II) können bei dem erfindungsgemäßen Verfahren gegebenenfalls ohne Isolierung direkt weiter umgesetzt werden.

Die bei der Herstellung der Oxirane der Formel (II) nach den obigen Verfahren als Ausgangsstoffe benötigten Ketone der Formel (IV) sind bekannt oder lassen sich nach prinzipiell bekannten Methoden in einfacher Weise herstellen (vgl. EP-OS 0 054 865 und DE-OS 3 222 220).

Die außerdem für das erfindungsgemäße Verfahren als Ausgangsstoffe benötigten Mercaptane sind durch die Formel (III) allgemein definiert.

In dieser Formel steht R vorzugsweise für die Bedeutungen, die bereits im Zusammenhang mit der Beschreibung der erfindungsgemäßen Stoffe der Formel (I) bevorzugt für R genannt wurden.

Die Mercaptane der Formel (III) sind allgemein bekannte Verbindungen der organischen Chemie.

Als Verdünnungsmittel kommen für das erfindungsgemäße Verfahren zur Herstellung von mercapto-substituierten Hydroxyethyl-(triazol-1-yl)-Derivaten der Formel (I) alle inerten organischen Solventien infrage. Vorzugsweise verwendbar sind Alkohole, wie Ethanol und Methoxyethanol; Ketone, wie z.B. 2-Butanon; Nitrile, wie z.B. Acetonitril; Ester, wie z.B. Essigester; Ether, wie z.B. Dioxan; aromatische Kohlenwasserstoffe, wie z.B. Benzol und Toluol; oder Amide, wie z.B. Dimethylformamid.

Als Säurebindemittel kommen für die erfindungsgemäße Umsetzung alle üblicherweise verwendbaren anorganischen und organischen Basen in Betracht. Hierzu gehören vorzugsweise Alkalicarbonate, wie z.B. Natrium-und Kaliumcarbonat; Alkalihydroxide,wie z.B. Natriumhydroxid; Alkalialkoholate, wie z.B. Natrium- und Kalium-methylat und -ethylat; Alkalihydride, wie z.B. Natriumhydrid; sowie niedere tertiäre Alkylamine, Cycloalkylamine und Aralkylamine, wie insbesondere Triethylamin.

Die Reaktionstemperaturen können bei der Durchführung des erfindungsgemäßen Verfahrens in einem größeren Bereich variiert werden. Im allgemeinen arbeitet man bei Temperaturen zwischen 0 und 200°C, vorzugsweise zwischen 60 und 150°C.

Bei der Durchführung des erfindungsgemäßen Verfahrens setzt man auf 1 Mol Oxiran der Formel (II) vorzugsweise 1 bis 2 Mol Mercaptan der Formel (III) und gegebenenfalls 1 bis 2 Mol Säurebindemittel ein. Die Isolierung der Endprodukte erfolgt in allgemein üblicher Weise.

Die nach dem erfindungsgemäßen Verfahren erhältlichen Verbindungen der Formel (I) können in Säureadditions-Salze bzw. Metallsalz-Komplexe überführt werden.

Zur Herstellung von Säureadditions-Salzen der Verbindungen der Formel (I) kommen vorzugsweise diejenigen Säuren in Frage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z.B. durch Lösen einer Verbindung der allgemeinen Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der allgemeinen Formel (I) kommen vorzugsweise diejenigen Salze von Metallen in Frage, die bereits weiter oben beschrieben wurden.

Die Metallsalz-Komplexe von Verbindungen der allgemeinen Formel (I) können in einfacher Weise nach üblichen Verfahren erhalten werden, so z.B. durch Lösen des Metallsalzes in Alkohol, z.B. Ethanol, und Hinzufügen zu Verbindungen der allgemeinen Formel (I). Man kann Metall salz-Komplexe in bekannter Weise, z.B. durch Abfiltrieren, isolieren und gegebenenfalls durch Umkristallisation reinigen.

Die erfindungsgemäßen Wirkstoffe weisen eine starke mikrobizide Wirkung auf und können als Fungizide eingesetzt werden.

Fungizide werden im Pflanzenschutz eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie Xanthomonas oryzae;
Pseudomonas-Arten, wie Pseudomonas lachrymans;
Erwinia-Arten, wie Erwinia amylovora;
Pythium-Arten, wie Pythium ultimum;
Phytophthora-Arten, wie Phytophthora infestans;
Pseudoperonospora-Arten, wie Pseudoperonospora humuli oder Pseudoperonospora cubense;
Plasmopara-Arten, wie Plasmopara viticola;
Peronospora-Arten, wie Peronospora pisi oder P. brassicae;
Erysiphe-Arten, wie Erysiphe graminis;
Sphaerotheca-Arten, wie Sphaerotheca fuliginea;
Podosphaera-Arten, wie Podosphaera leucotricha;
Venturia-Arten, wie Venturia inaequalis;
Pyrenophora-Arten, wie Pyrenophora teres oder P. graminea;
(Konidienform: Drechslera, Syn: Helminthosporium);
Cochliobolus-Arten, wie Cochliobolus sativus;
(Konidienform: Drechslera, Syn: Helminthosporium);
Uromyces-Arten, wie Uromyces appendiculatus;
Puccinia-Arten, wie Puccinia recondita;
Tilletia-Arten, wie Tilletia caries;
Ustilago-Arten, wie Ustilago nuda oder Ustilago avenae;
Pellicularia-Arten, wie Pellicularia sasakii;
Pyricularia-Arten, wie Pyricularia oryzae;
Fusarium-Arten, wie Fusarium culmorum;
Botrytis-Arten, wie Botrytis cinerea;
Septoria-Arten, wie Septoria nodorum;
Leptosphaeria-Arten, wie Leptosphaeria nodorum;
Cercospora-Arten, wie Cercospora canescens;
Alternaria-Arten, wie Alternaria brassicae;
Pseudocercosporella-Arten, wie Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz-und Saatgut und des Bodens.

Die erfindungsgemäßen Wirkstoffe können mit besonders gutem Erfolg protektiv gegen Leptosphaeria nodorum bei Weizen und Botrytis bei Bohnen sowie als Saatgutbehandlungsmittel bei Weizen gegen Fusarium culmorum eingesetzt werden.

Hervorzuheben ist, daß die erfindungsgemäßen Stoffe nicht nur protektiv, sondern auch systemisch eingesetzt werden können zur Bekämpfung von Mehltau und Pyrenophora teres an Getreide sowie gegen Pyricularia oryzae an Reis.

Außerdem besitzen die erfindungsgemäßen Wirkstoffe auch pflanzenwuchsregulierende Eigenschaften.

Die erfindungsgemäßen Wirkstoffe besitzen darüber hinaus auch parasitizide Wirksamkeit und eignen sich zur Bekämpfung von Insekten, Milben, Zecken usw. auf dem Gebiet der Tierhaltung und Viehzucht, wobei durch die Bekämpfung der Schädlinge bessere Ergebnisse, z.B. höhere Milchleistungen, höheres Gewicht, schöneres Tierfell, längere Lebensdauer usw. erreicht werden können.

Die Anwendung der erfindungsgemäßen Wirkstoffe geschieht auf diesem Gebiet in bekannter Weise, wie durch äußerliche Anwendung in Form beispielsweise des Tauchens (Dippen), Sprühens (Sprayen), Aufgießen (pour-on and spot-on) und des Einpuderns.

Die Wirkstoffe eignen sich besonders zur Bekämpfung von Ektoparasiten wie z. B. Psoroptes ovis und Boophilus microplus.

Die Wirkstoffe können in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind. z.B. Aerosol-Treibgase, wie Halogenkohlenwasserstoffe sowie Butan, Propan, Stickstoff und Kohlendioxid;
als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder -schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo-und Metallphthalocyaninfarbstoffe und Spurennährstoffe wie Salze von Eisen, Mangan, Bor, Kupfer, Kobalt, Molybdän und Zink verwendet werden.
Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie Mischungen mit Düngemitteln und anderen Wachstumsgulatoren.

Die Wirkstoffe können als solche, in Form ihrer Formulierungen oder den daraus bereiteten Anwendungsformen wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

Die Herstellung und die Verwendung der erfindungsgemäßen Wirkstoffe geht aus den folgenden Beispielen hervor.

Herstellungsbeispiele

Beispiel 1

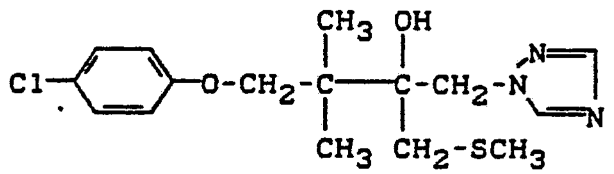

(I-1)

Zu 18 g (0,042 Mol) 2-(4-Chlor-phenoxy-tert.-butyl)-2-(1,2,4-triazol-1-yl-methyl)-oxiran in 80 ml Ethanol werden unter Rühren 1,8 g Natriumhydroxid in 80 ml Ethanol gegeben. Der Ansatz wird gekühlt und bei -15°C werden 2,4 g (0,055 Mol) Methylmercaptan eingeleitet. Anschließend läßt man das Reaktionsgemisch langsam auf Raumtemperatur kommen und rührt weitere 16 Stunden. Der Ansatz wird eingeengt, das verbleibende Öl in 350 ml Dichlormethan aufgenommen, mit zweimal 500 ml Wasser gewaschen, über Natriumsulfat getrocknet und eingeengt. Der Rückstand wird über eine Kieselgelsäule (Laufmittel: Cyclohexan/Essigester1:1) gereinigt.

Man erhält 12 g (80 % der Theorie) 2-(Methylthiomethyl)-3,3-dimethyl-4-(4-chlor-phenoxy)-1-(1,2,4-triazol-1-yl)-butan-2-ol als zähes Öl. Die Struktur der Verbindung wird durch das Kernresonanzspektrum belegt.

Herstellung von Ausgangsprodukten

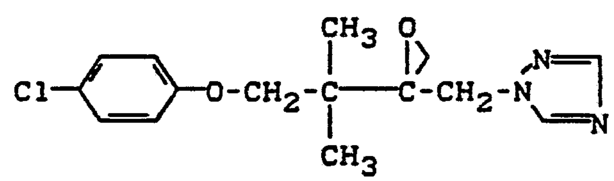

(II-1)

In eine Lösung von 170 ml absolutem Dimethylsulfoxid und 78,4 g Kalium-tert.-butylat werden 154 g (0,7 Mol) Trimethylsulfoxoniumjodid gegeben. Der Ansatz wird 5 Stunden gerührt, danach tropfenweise mit 182 g (0,7 Mol) 1-(1,2,4-Triazol-1-yl)-4-(4-chlor-phenoxy)-3,3-dimethylbutan-2-on versetzt und 16 Stunden bei 45°C gerührt. Nach der Zugabe von 300 ml Wasser wird die organische Phase abgetrennt und die Wasser-Phase einmal mit 500 ml Dichlormethan extrahiert. Die organischen Phasen werden vereinigt und zweimal mit 1200 ml Wasser gewaschen. Nach dem Trocknen über Natriumsulfat wird das Verdünnungsmittel abgezogen und der Rückstand im Hochvakuum entgast.

Man erhält 187 g (86,8 % der Theorie) 2-(4-Chlorphenoxy-tert.-butyl)-2-(1,2,4-triazol-1-yl-methyl)-oxiran als Öl.

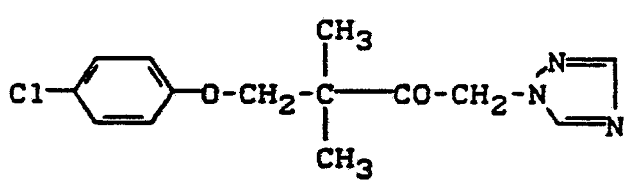

(IV-1)

Zu einem Gemisch aus 138 g (1 Mol) Kaliumcarbonat und 50,4 g (0,73 Mol) 1,2,4-Triazol in 800 ml absolutem siedenden Aceton werden 222 g (0,73 Mol) 1-Brom-4-(4-Chlor-phenoxy)-3,3-dimethyl-butan-2-on, gelöst in Aceton, unter Rühren zugetropft.

Man erwärmt 5 Stunden unter Rückfluß, kühlt auf 0 bis 10°C ab, filtriert von Salz ab und engt das Filtrat unter vermindertem Druck ein. Der Rückstand wird in 800 ml Dichlormethan aufgenommen und zweimal mit 1300 ml Wasser gewaschen. Die organische Phase wird über Natriumsulfat getrocknet und eingeengt.

Man erhält 182 g (85 % der Theorie) 1-(1,2,4-Triazol-1-yl)-4-(4-chlor-phenoxy)-3,3-dimethylbutan-2-on in Form farbloser Kristalle vom Schmelzpunkt 88-90°C.

In analoger Weise zu der im Beispiel 1 beschriebenen Methode und unter Berücksichtigung der Angaben zu dem erfindungsgemäßen Verfahren werden die nachfolgend aufgeführten Verbindungen der Formel

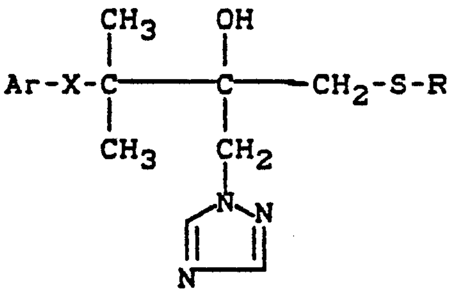

(I)

erhalten:

Tabelle 1

| Bsp. Nr. | Verb. Nr. | Ar | X | R | Schmelzpunkt [°C] bzw. Brechungsindex [$n_D^{20}$] |
|---|---|---|---|---|---|
| 2 | I-2 | 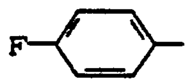 | -$CH_2$ | $C_2H_5$ | Öl |
| 3 | I-3 | 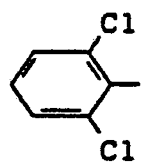 | -O-$CH_2$ | n$C_3H_7$ | Öl |
| 4 | I-4 | 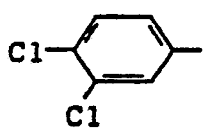 | -O-$CH_2$ | $C_2H_5$ | 85 |
| 5 | I-5 | 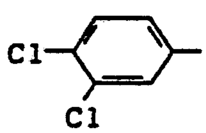 | -O-$CH_2$ | i$C_3H_7$ | 94-97 |
| 6 | I-6 | 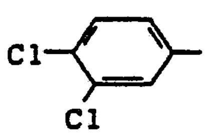 | -O-$CH_2$ | 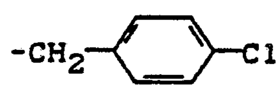 | 92-94 |
| 7 | I-7 | 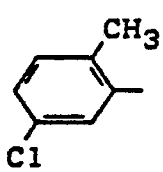 | -O-$CH_2$ | $C_2H_5$ | Öl |
| 8 | I-8 | 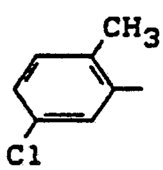 | -O-$CH_2$ | n$C_4H_9$ | Öl |
| 9 | I-9 | 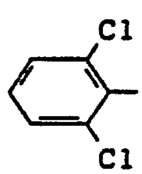 | -O-$CH_2$ | n$C_4H_9$ | Öl |

<u>Tabelle 1</u> (Fortsetzung)

| Bsp. Nr. | Verb. Nr. | Ar | X | R | Schmelzpunkt [°C] bzw. Brechungsindex [$n_D^{20}$] |
|---|---|---|---|---|---|
| 10 | I-10 | 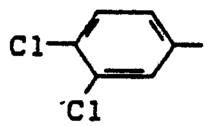 | -O-$CH_2$ | $nC_3H_7$ | 71-73 |
| 11 | I-11 | 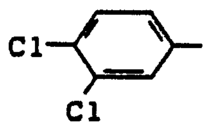 | -O-$CH_2$ | $nC_4H_9$ | Öl |
| 12 | I-12 | 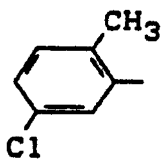 | -O-$CH_2$ | $nC_3H_7$ | Öl |
| 13 | I-13 | 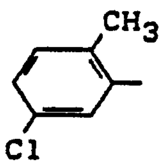 | -O-$CH_2$ | $iC_3H_7$ | 86-99 |
| 14 | I-14 | 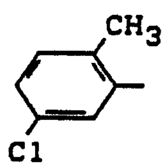 | -O-$CH_2$ | -$CH_2$- 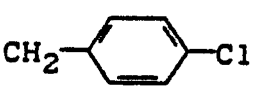 | Öl |
| 15 | I-15 | 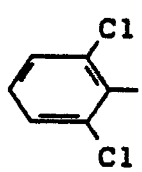 | -O-$CH_2$ | $C_2H_5$ | Öl |
| 16 | I-16 | 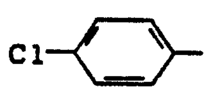 | -O-$CH_2$ | -$CH_2$ 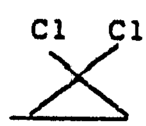 | Öl |

<u>Tabelle 1</u> (Fortsetzung)

| Bsp. Nr. | Verb. Nr. | Ar | X | R | Schmelzpunkt [°C] bzw. Brechungsindex [$n_D^{20}$] |
|---|---|---|---|---|---|
| 17 | I-17 | 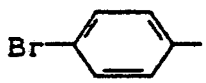 | $-O-CH_2$ | 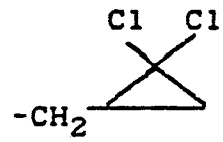 | Öl |
| 18 | I-18 | 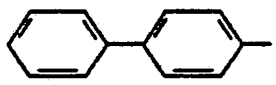 | $-O-CH_2$ | 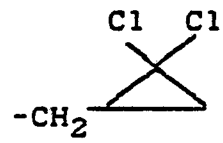 | Öl |
| 19 | I-19 | 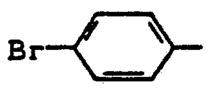 | $-O-CH_2$ | $C_2H_5$ | Öl |
| 20 | I-20 | 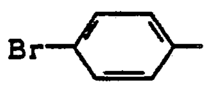 | $-O-CH_2$ | $nC_3H_7$ | Öl |
| 21 | I-21 | 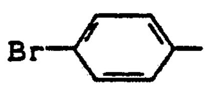 | $-O-CH_2$ | $iC_3H_7$ | Öl |
| 22 | I-22 | 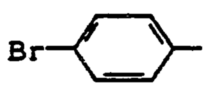 | $-O-CH_2$ | $nC_4H_9$ | Öl |
| 23 | I-23 | 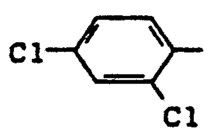 | $-O-CH_2$ | $C_2H_5$ | Öl |
| 24 | I-24 | 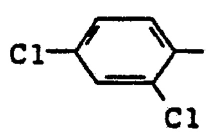 | $-O-CH_2$ | $nC_3H_7$ | Öl |
| 25 | I-25 | 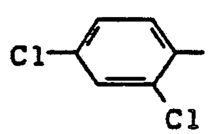 | $-O-CH_2$ | $nC_4H_9$ | Öl |

Tabelle 1 (Fortsetzung)

| Bsp. Nr. | Verb. Nr. | Ar | X | R | Schmelzpunkt [°C] bzw. Brechungsindex [$n_D^{20}$] |
|---|---|---|---|---|---|
| 26 | I-26 | 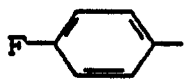 | $-CH_2$ | $-CH_2$ 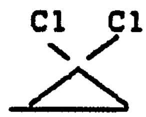 | Öl |
| 27 | I-27 | 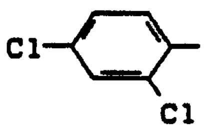 | $-O-CH_2$ | $iC_3H_7$ | Öl |
| 28 | I-28 | 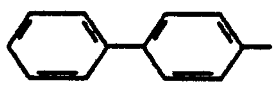 | $-O-CH_2$ | $C_2H_5$ | Öl |
| 29 | I-29 | 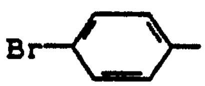 | $-O-CH_2$ | $CH_3$ | 69-71 |
| 30 | I-30 | 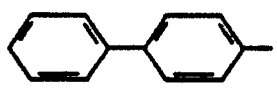 | $-O-CH_2$ | $CH_3$ | 120-123 |
| 31 | I-31 | 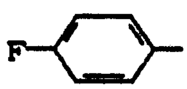 | $-CH_2$ | $CH_3$ | 135 |
| 32 | I-32 | 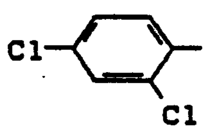 | $-O-CH_2$ | $CH_3$ | Öl |
| 33 | I-33 | 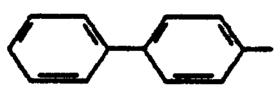 | $-O-CH_2$ | $nC_4H_9$ | Öl |
| 34 | I-34 | 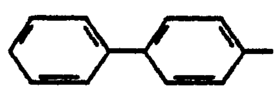 | $-O-CH_2$ | $nC_3H_7$ | Öl |

Tabelle 1 (Fortsetzung)

| Bsp. Nr. | Verb. Nr. | Ar | X | R | Schmelzpunkt [°C] bzw. Brechungsindex [$n_D^{20}$] |
|---|---|---|---|---|---|
| 35 | I-35 | 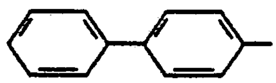 | $O-CH_2$ | $iC_3H_7$ | Öl |
| 36 | I-36 | 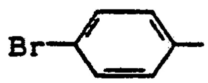 | $-CH_2$ | $-CH_2-CH=CH_2$ | 108 |
| 37 | I-37 | 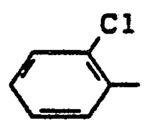 | $-CH_2$ | $iC_3H_7$ | Öl |
| 38 | I-38 | 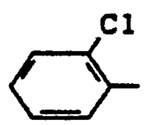 | $-CH_2$ | $-CH_2-CH=CH_2$ | Öl |
| 39 | I-39 | 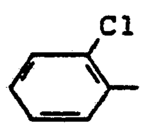 | $-CH_2$ | $C_2H_5$ | Öl |
| 40 | I-40 | 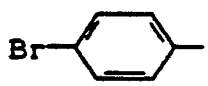 | $-CH_2$ | $iC_3H_7$ | 80-81 |
| 41 | I-41 | 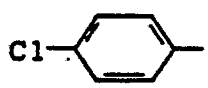 | $-CH_2$ | $nC_3H_7$ | Öl |
| 42 | I-42 | 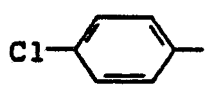 | $-CH_2$ | $CH_3$ | 146 |
| 43 | I-43 | 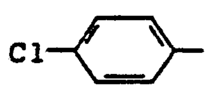 | $-CH_2$ | $iC_4H_9$ | Öl |
| 44 | I-44 | 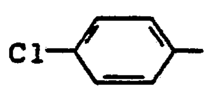 | $-CH_2$ | $-CH_2-CH=CH_2$ | Öl |

Tabelle 1 (Fortsetzung)

| Bsp. Nr. | Verb. Nr. | Ar | X | R | Schmelzpunkt [°C] bzw. Brechungsindex [$n_D^{20}$] |
|---|---|---|---|---|---|
| 45 | I-45 | 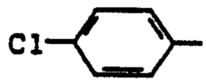 | $CH_2$ | $C_2H_5$ | 82-83 |
| 46 | I-46 | 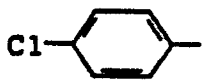 | $CH_2$ | $iC_3H_7$ | Harz |
| 47 | I-47 | 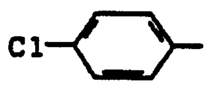 | $CH_2$ | $nC_4H_9$ | Harz |
| 48 | I-48 | 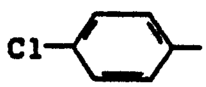 | $CH_2$ | $nC_6H_{13}$ | Harz |
| 49 | I-49 | 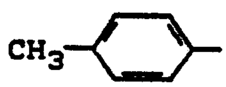 | $CH_2$ | $nC_3H_7$ | Öl |
| 50 | I-50 | 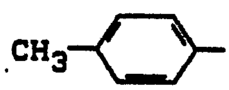 | $CH_2$ | $C_2H_5$ | 102-103 |
| 51 | I-51 | 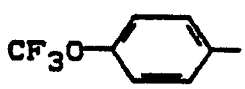 | $CH_2$ | $nC_3H_7$ | Öl |
| 52 | I-52 | 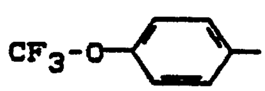 | $CH_2$ | $C_2H_5$ | Öl |
| 53 | I-53 | 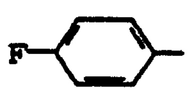 | $CH_2$ | $nC_3H_7$ | Öl |
| 54 | I-54 | 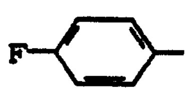 | $CH_2$ | $nC_4H_9$ | Öl |
| 55 | I-55 | 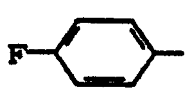 | $CH_2$ | $iC_3H_7$ | 76 |

Tabelle 1 (Fortsetzung)

| Bsp. Nr. | Verb. Nr. | Ar | X | R | Schmelzpunkt [°C] bzw. Brechungsindex [$n_D^{20}$] |
|---|---|---|---|---|---|
| 56 | I-56 | 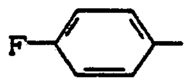 | $CH_2$ | H | Öl |
| 57 | I-57 | 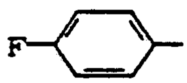 | $CH_2$ | $nC_6H_{13}$ | Öl |
| 58 | I-58 | 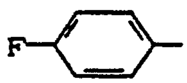 | $CH_2$ | 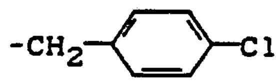 | Öl |
| 59 | I-59 | 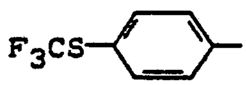 | $CH_2$ | $C_2H_5$ | Öl |
| 60 | I-60 | 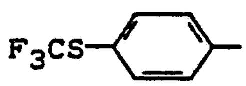 | $CH_2$ | $nC_3H_7$ | Öl |
| 61 | I-61 | 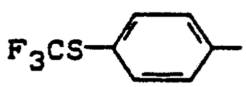 | $CH_2$ | $nC_4H_9$ | Öl |
| 62 | I-62 | 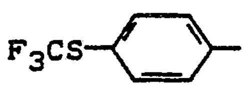 | $CH_2$ | $iC_3H_7$ | Öl |
| 63 | I-63 | 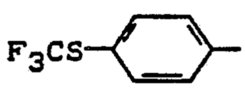 | $CH_2$ | H | Öl |
| 64 | I-64 | 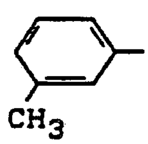 | $CH_2$ | $C_2H_5$ | 78 |
| 65 | I-65 | 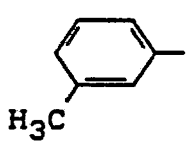 | $CH_2$ | $iC_3H_7$ | Öl |

Tabelle 1 (Fortsetzung)

| Bsp. Nr. | Verb. Nr. | Ar | X | R | Schmelzpunkt [°C] bzw. Brechungsindex [$n_D^{20}$] |
|---|---|---|---|---|---|
| 66 | I-66 | 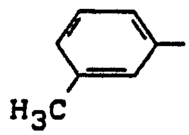 | $CH_2$ | $nC_4H_9$ | Öl |
| 67 | I-67 | 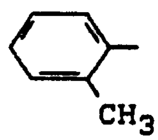 | $CH_2$ | $C_2H_5$ | Öl |
| 68 | I-68 | 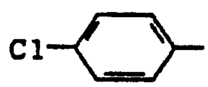 | $OCH_2$ | $C_2H_5$ | Öl |
| 69 | I-69 | 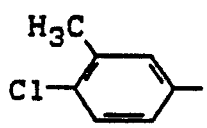 | $OCH_2$ | $C_2H_5$ | Öl |
| 70 | I-70 | 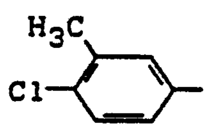 | $OCH_2$ | $nC_3H_7$ | Öl |
| 71 | I-71 | 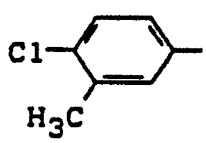 | $OCH_2$ | $nC_4H_9$ | Öl |
| 72 | I-72 | 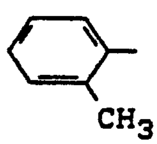 | $CH_2$ | $nC_3H_7$ | Öl |
| 73 | I-73 | 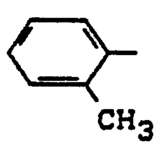 | $CH_2$ | $iC_3H_7$ | Öl |

Tabelle 1 (Fortsetzung)

| Bsp. Nr. | Verb. Nr. | Ar | X | R | Schmelzpunkt [°C] bzw. Brechungsindex [$n_D^{20}$] |
|---|---|---|---|---|---|
| 74 | I-74 | 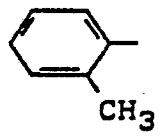 | $CH_2$ | $nC_4H_9$ | Öl |
| 75 | I-75 | 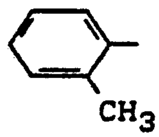 | $CH_2$ | 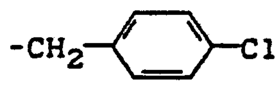 | Öl |
| 76 | I-76 | 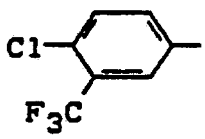 | $CH_2$ | $nC_4H_9$ | Öl |
| 77 | I-77 | 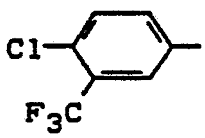 | $CH_2$ | $C_2H_5$ | Öl |
| 78 | I-78 | 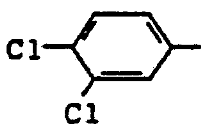 | $CH_2$ | $C_2H_5$ | Öl |
| 79 | I-79 | 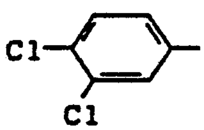 | $CH_2$ | $nC_3H_7$ | Öl |
| 80 | I-80 | 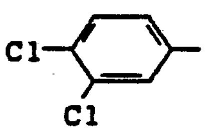 | $CH_2$ | $iC_3H_7$ | Öl |
| 81 | I-81 | 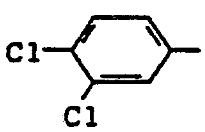 | $CH_2$ | $nC_4H_9$ | Öl |

Tabelle 1 (Fortsetzung)

| Bsp. Nr. | Verb. Nr. | Ar | X | R | Schmelzpunkt [°C] bzw. Brechungsindex [$n_D^{20}$] |
|---|---|---|---|---|---|
| 82 | I-82 | 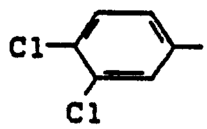 | $CH_2$ | 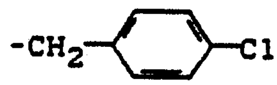 | Öl |
| 83 | I-83 | 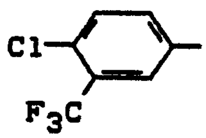 | $CH_2$ | $iC_3H_7$ | Öl |
| 84 | I-84 | 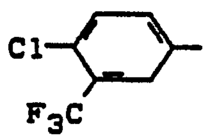 | $CH_2$ | $nC_3H_7$ | Öl |
| 85 | I-85 | 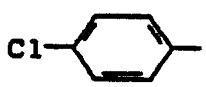 | $OCH_2$ | $nC_3H_7$ | Öl |
| 86 | I-86 | 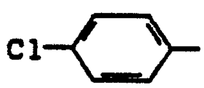 | $OCH_2$ | $iC_3H_7$ | Öl |
| 87 | I-87 | 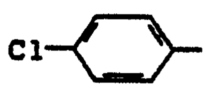 | $OCH_2$ | $nC_4H_9$ | Öl |
| 88 | I-88 | 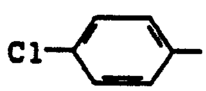 | $OCH_2$ | $nC_6H_{13}$ | Öl |
| 89 | I-89 | 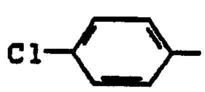 | $OCH_2-CH_2$ | $nC_3H_7$ | Öl |
| 90 | I-90 | 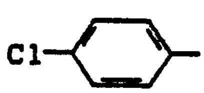 | $OCH_2-CH_2$ | $iC_3H_7$ | Öl |
| 91 | I-91 | 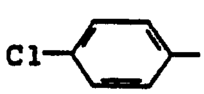 | $OCH_2-CH_2$ | $C_2H_5$ | Öl |

Tabelle 1 (Fortsetzung)

| Bsp. Nr. | Verb. Nr. | Ar | X | R | Schmelzpunkt [°C] bzw. Brechungsindex [$n_D^{20}$] |
|---|---|---|---|---|---|
| 92 | I-92 | 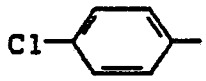 | $OCH_2-CH_2$ | $-CH_2-CH=CH_2$ | Öl |
| 93 | I-93 | 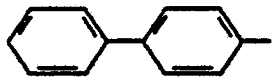 | O | 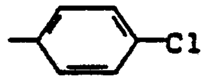 | Harz |
| 94 | I-94 | 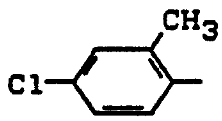 | O | 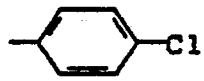 | Harz |
| 95 | I-95 | 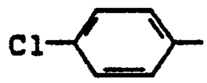 | O | $C_2H_5$ | 65 |
| 96 | I-96 | 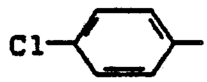 | O | 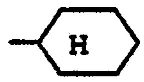 | 1.5405 |
| 97 | I-97 | 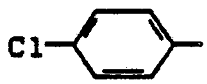 | O | $nC_3H_7$ | 1.5398 |
| 98 | I-98 | 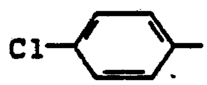 | O | $iC_3H_7$ | 1.5320 |
| 99 | I-99 | 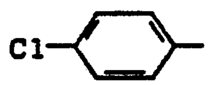 | O | $nC_6H_{13}$ | Öl |

Nach der im Beispiel 1 angegebenen Methode werden auch die in der folgenden Tabelle 2 aufgeführten Oxirane der Formel

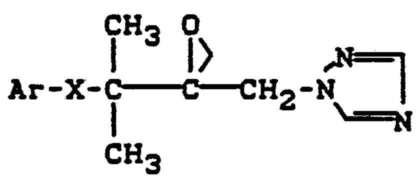

(II)

hergestellt.

Tabelle 2

| Bsp. Nr. | Verb. Nr. | Ar | X | Schmelzpunkt [°C] bzw. Brechungsindex [$n_D^{20}$] |
|---|---|---|---|---|
| 100 | II-2 | 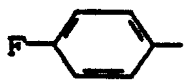 | $-CH_2$ | Öl |
| 101 | II-3 | 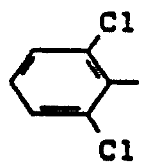 | $-OCH_2$ | Öl |
| 102 | II-4 | 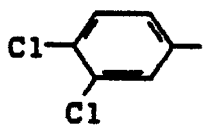 | $-OCH_2$ | Öl |
| 103 | II-5 | 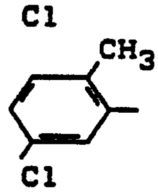 | $-OCH_2$ | Öl |
| 104 | II-6 | 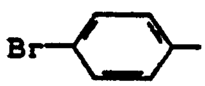 | $-OCH_2$ | Öl |
| 105 | II-7 | 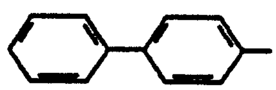 | $-OCH_2$ | Öl |
| 106 | II-8 | 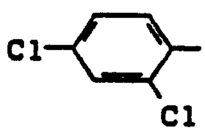 | $-OCH_2$ | Öl |

<u>Tabelle 2</u> (Fortsetzung)

| Bsp. Nr. | Verb. Nr. | Ar | X | Schmelzpunkt [°C] bzw. Brechungsindex [$n_D^{20}$] |
|---|---|---|---|---|
| 107 | II-9 | 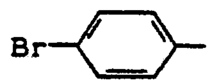 | $CH_2$ | Öl |
| 108 | II-10 | 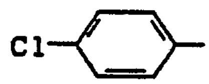 | $-CH_2-$ | Öl |
| 109 | II-11 | 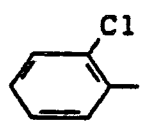 | $-CH_2-$ | Öl |
| 110 | II-12 | 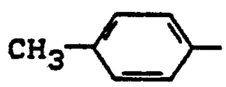 | $-CH_2-$ | Öl |
| 111 | II-13 | 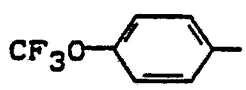 | $-CH_2-$ | Öl |
| 112 | II-14 | 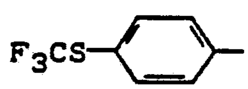 | $-CH_2-$ | Öl |
| 113 | II-15 | 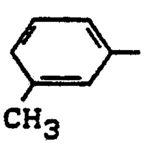 | $-CH_2-$ | Öl |
| 114 | II-16 | 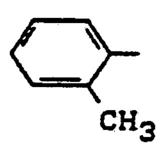 | $-CH_2-$ | Öl |
| 115 | II-17 | 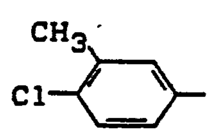 | $-OCH_2$ | Öl |

Tabelle 2 (Fortsetzung)

| Bsp. Nr. | Verb. Nr. | Ar | X | Schmelzpunkt [°C] bzw. Brechungsindex [$n_D^{20}$] |
|---|---|---|---|---|
| 116 | II-18 | 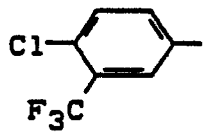 | $-CH_2-$ | Öl |
| 117 | II-19 | 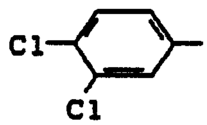 | $-CH_2-$ | Öl |
| 118 | II-20 | 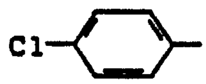 | $-OCH_2CH_2$ | Öl |
| 119 | II-21 | 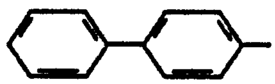 | O | Öl |
| 120 | II-22 | 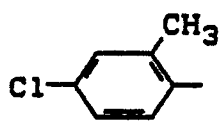 | O | Öl |
| 121 | II-23 | 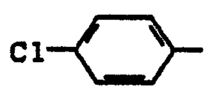 | O | Öl |
| 122 | II-24 | 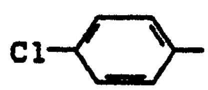 | O | Öl |

In analoger Weise zu der im Beispiel 1 beschriebenen Methode und unter Berücksichtigung der Angaben zu dem erfindungsgemäßen Verfahren werden die nachfolgend aufgeführten Verbindungen der Formel

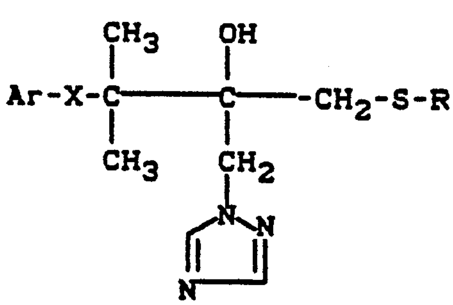

(I)

erhalten:

Tabelle 3

| Bsp. Nr. | Verb. Nr. | Ar | X | R | Schmelzpunkt [°C] bzw. Brechungsindex [$n_D^{20}$] |
|---|---|---|---|---|---|
| 123 | I-100 | 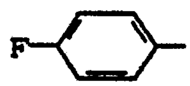 | $-CH_2$ | $-CH_2-CH=CH_2$ | 74-76 |
| 124 | I-101 | 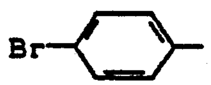 | $-OCH_2-$ | $-CH_2-CH=CH_2$ | Öl |
| 125 | I-102 | 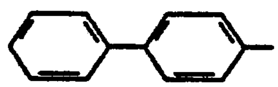 | $-O-CH_2-$ | $-CH_2-CH=CH_2$ | Öl |
| 126 | I-103 | 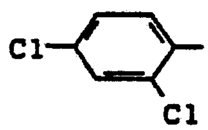 | $-O-CH_2-$ | $-CH_2-CH=CH_2$ | Öl |
| 127 | I-104 | 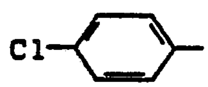 | $-O-CH_2-$ | $-CH_2-CH=CH_2$ | Öl |
| 128 | I-105 | 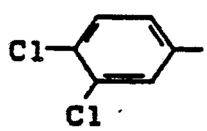 | $-O-CH_2-$ | $-CH_2-CH=CH_2$ | Öl |
| 129 | I-106 | 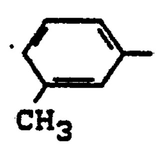 | $-CH_2-$ | $-CH_2-CH=CH_2$ | 66-68 |
| 130 | I-107 | 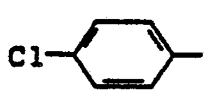 | -O- | $n-C_4H_9$ | 1,5349 |
| 131 | I-108 | 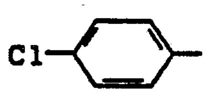 | -O- | $i-C_4H_9$ | 1,5310 |

Verwendungsbeispiele:

In den folgenden Verwendungsbeispielen werden die nachstehend angegebenen Substanzen als Vergleichsverbindungen eingesetzt.

(A) =

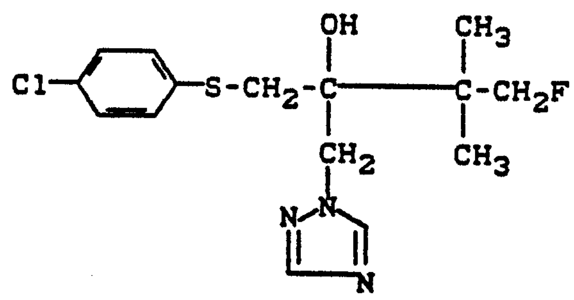

(B) =

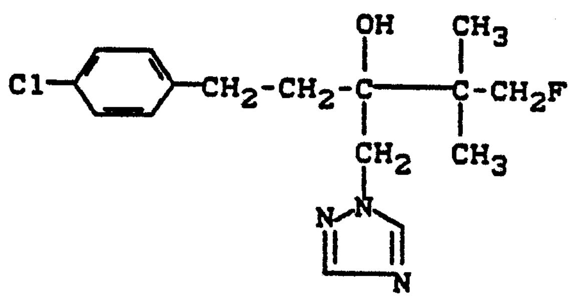

(C) =

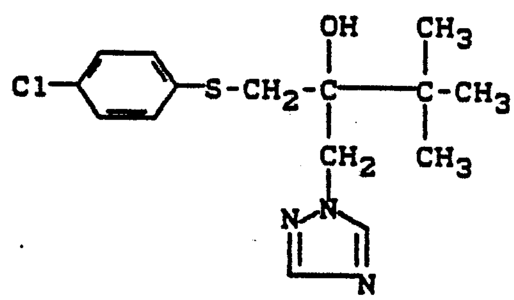

(D) =

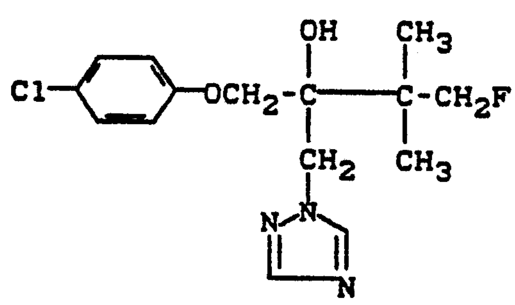

Beispiel A

Botrytis-Test (Bohne)/protektiv Lösungsmittel: 4,7 Gewichtsteile Aceton
Emulgator: 0,3 Gewichtsteile Alkyl-aryl-polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten Kammer bei 20°C aufgestellt. 3 Tage nach der Inokulation wird die Größe der Befallsflecken auf den Blättern ausgewertet.

In diesem Test zeigen die erfindungsgemäßen Stoffe (I-2), (I-43), (I-44), (I-45), (I-50) und (I-68) eine bessere Wirksamkeit als die Vergleichssubstanz (A).

Beispiel B

Leptosphaeria nodorum-Test (Weizen) / protektiv Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Leptosphaeria nodorum besprüht. Die Pflanzen verbleiben 48 Stunden bei 20°C und 100 % relativer Luftfeuchtigkeit in einer Inkubationskabine.

Die Pflanzen werden in einem Gewächshaus bei einer Temperatur von ca. 15°C und einer relativen Luftfeuchtigkeit von ca. 80 % aufgestellt.

10 Tage nach der Inokulation erfolgt die Auswertung.

In diesem Test zeigen die erfindungsgemäßen Stoffe (I-2), (I-41), (I-46), (I-47), (I-48), (I-53), (I-54), (I-55), (I-58), (I-59), (I-60), (I-61) und (I-68) eine bessere Wirksamkeit als die Vergleichssubstanz (B).

Beispiel C

Fusarium culmorum-Test (Weizen) / Saatgutbehandlung

Die Anwendung der Wirkstoffe erfolgt als Trockenbeizmittel. Sie werden zubereitet durch Abstrecken des jeweiligen Wirkstoffes mit Gesteinsmehl zu einer feinpulvrigen Mischung, die eine gleichmäßige Verteilung auf der Saatgutoberfläche gewährleistet.

Zur Beizung schüttelt man das infizierte Saatgut 3 Minuten lang mit dem Beizmittel in einer verschlossenen Glasflasche.

Den Weizen sät man mit 2 x 100 Korn 1 cm tief in eine Standarderde und kultiviert ihn im Gewächshaus bei einer Temperatur von ca. 18°C in Saatkästen, die täglich 15 Stunden dem Licht ausgesetzt werden.

Ca. 3 Wochen nach der Aussaat erfolgt die Auswertung der Pflanzen auf Symptome.

In diesem Test zeigt der erfindungsgemäße Wirkstoff (I-46) eine bessere Wirksamkeit als die Vergleichssubstanz (B).

Beispiel D

Test mit Psoroptes ovis Lösungsmittel:
35 Gewichtsteile Ethylenglykolmonomethylether
35 Gewichtsteile Nonylphenolpolyglykolether

Zur Herstellung einer zweckmäßigen Wirkstoffzubereitung vermischt man drei Gewichtsteile Wirkstoff mit sieben Gewichtsteilen des oben angegebenen Lösungsmittel-Gemisches und verdünnt das so erhaltene Konzentrat mit Wasser auf die gewünschte Konzentration.

Etwa 10 - 25 Psoroptes ovis werden in 1 ml der zu testenden Wirkstoffzubereitung gebracht, die in Tablettennester einer Tiefziehverpackung pipettiert wurden. Nach 24 Stunden wird der Abtötungsgrad bestimmt.

In diesem Test zeigen die erfindungsgemäßen Wirkstoffe (I-41), (I-57) und (I-60) eine bessere Wirksamkeit als die Vergleichssubstanzen (C) und (D).

**Ansprüche**

1. Mercapto-substituierte Hydroxyethyl-(triazol-1-yl)-Derivate der Formel

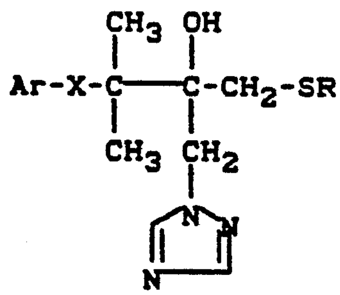

(I)

in welcher

Ar für gegebenenfalls substituiertes Aryl steht,

X für Sauerstoff, Schwefel, eine direkte Bindung sowie für die Gruppierungen $-CH_2-$, $-CH_2-CH_2-$, $-O-CH_2-$, $-SCH_2-$, $-O-CH_2-CH_2-$und $-S-CH_2-CH_2-$steht, wobei das Heteroatom mit dem Aryl-Rest verbunden ist, wenn X für $-OCH_2-$, $-SCH_2-$, $-O-CH_2-CH_2-$oder $-S-CH_2-CH_2-$steht, und

R für Wasserstoff, gegebenenfalls substituiertes Alkyl, gegebenenfalls substituiertes Alkenyl, gegebenenfalls substituiertes Alkinyl, gegebenenfalls substituiertes Cycloalkyl, gegebenenfalls substituiertes Cycloalkylalkyl, gegebenenfalls substituiertes Aralkyl oder für gegebenenfalls substituiertes Phenyl steht,

sowie deren Säureadditions-Salze und Metallsalz-Komplexe.

2. Mercapto-substituierte Hydroxyethyl-(triazol-1-yl)-Derivate der Formel (I) gemäß Anspruch 1, in denen

Ar für Aryl mit 6 bis 10 Kohlenstoffatomen steht, welches einfach oder mehrfach, gleichartig oder verschieden substituiert sein kann durch Halogen, gegebenenfalls substituiertes Alkyl, gegebenenfalls substituiertes Alkoxy, gegebenenfalls substituiertes Alkylthio, gegebenenfalls substituiertes Alkenyl, gegebenenfalls substituiertes Alkinyl, gegebenenfalls substituiertes Phenyl, gegebenenfalls substituiertes Phenoxy, gegebenenfalls substituiertes Phenylalkyl, Nitro, Cyano, Formyl, Alkylcarbonyl und/oder von Formyl oder Alkylcarbonyl abgeleitete Oxime, Ketale und Hydrazone,

X für Sauerstoff, Schwefel, eine direkte Bindung sowie für die Gruppierungen $-CH_2-$, $-CH_2-CH_2-$, $-O-CH_2-$, $-SCH_2-$, $-O-CH_2-CH_2-$und $-S-CH_2-CH_2-$steht, wobei das Heteroatom mit dem Aryl-Rest verbunden ist, wenn X für $-OCH_2-$, $-SCH_2-$, $-O-CH_2-$oder $-S-CH_2-CH_2-$steht, und

R für Wasserstoff, gegebenenfalls substituiertes, geradkettiges oder verzweigtes Alkyl mit 1 bis 12 Kohlenstoffatomen, gegebenenfalls substituiertes Alkenyl mit 2 bis 12 Kohlen stoffatomen, gegebenenfalls substituiertes Alkinyl mit 3 bis 8 Kohlenstoffatomen, gegebenenfalls einfach oder zweifach, gleichartig oder verschieden durch Halogen und/oder Alkyl substituiertes Cycloalkyl mit 3 bis 6 Kohlenstoffatomen, gegebenenfalls einfach oder zweifach, gleichartig oder verschieden durch Halogen und/oder Alkyl substituiertes Cycloalkylalkyl mit 3 bis 6 Kohlenstoffatomen im Cycloalkylteil und 1 oder 2 Kohlenstoffatomen im Alkylteil oder für Aralkyl mit 6 bis 10 Kohlenstoffatomen im Arylteil und 1 bis 4 Kohlenstoffatomen im Alkylteil steht, wobei der Arylteil einfach oder mehrfach, gleichartig oder verschieden substituiert sein kann durch Halogen,

gegebenenfalls substituiertes Alkyl, gegebenenfalls substituiertes Alkoxy, gegebenenfalls substituiertes Alkylthio, gegebenenfalls substituiertes Alkenyl, gegebenenfalls substituiertes Alkinyl, gegebenenfalls substituiertes Phenyl, gegebenenfalls substituiertes Phenoxy, gegebenenfalls substituiertes Phenylalkyl, Nitro, Cyano, Formyl, Alkylcarbonyl und/oder von Formyl oder Alkylcarbonyl abgeleitete Oxime, Ketale und Hydrazone, oder

R für gegebenenfalls durch Halogen und/oder Alkyl substituiertes Phenyl steht.

3. Verfahren zur Herstellung von mercapto-substituierten Hydroxyethyl-(triazol-1-yl)-Derivaten der Formel

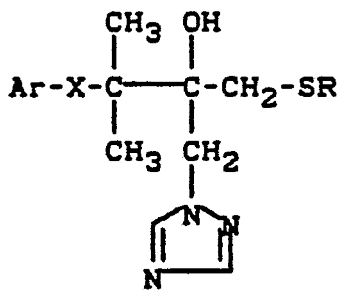

(I)

in welcher

Ar für gegebenenfalls substituiertes Aryl steht,

X für Sauerstoff, Schwefel, eine direkte Bindung sowie für die Gruppierungen $-CH_2-$, $-CH_2-CH_2-$, $-O-CH_2-$, $-SCH_2-$, $-O-CH_2-CH_2-$und $-S-CH_2-CH_2-$steht, wobei das Heteroatom mit dem Aryl-Rest verbunden ist, wenn X für $-OCH_2-$, $-SCH_2-$, $-O-CH_2-CH_2-$oder $-S-CH_2-CH_2$ steht, und

R für Wasserstoff, gegebenenfalls substituiertes Alkyl, gegebenenfalls substituiertes Alkenyl, gegebenenfalls substituiertes Alkinyl, gegebenenfalls substituiertes Cycloalkyl, gegebenenfalls substituiertes Cycloalkylalkyl, gegebenenfalls substituiertes Aralkyl oder für gegebenenfalls substituiertes Phenyl steht,

sowie deren Säureadditions-Salzen und Metallsalz-Komplexen, dadurch gekennzeichnet, daß man Oxirane der Formel

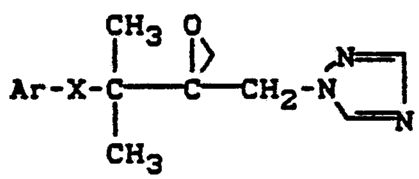

(II)

in welcher

Ar und X die oben angegebene Bedeutung haben,

mit Mercaptanen der Formel

R-SH (III)

in welcher

R die oben angegebene Bedeutung hat,

in Gegenwart eines Verdünnungsmittels und gegebenenfalls in Gegenwart eines Säurebindemittels umsetzt und gegebenenfalls anschließend an die so erhaltenen Verbindungen der Formel (I) eine Säure oder ein Metallsalz addiert.

4. Fungizide und parasitizide Mittel, gekennzeichnet durch einen Gehalt an mindestens einem mercapto-substituierten Hydroxyethyl-(triazol-1-yl)-Derivat der Formel (I) gemäß Anspruch 1 bzw. an einem Säureadditions-Salz oder Metallsalz-Komplex eines mercapto-substituierten Hydroxyethyl-(triazol-1-yl)-Derivates der Formel (I).

5. Verwendung von mercapto-substituierten Hydroxyethyl(triazol-1-yl)-Derivaten der Formel (I) gemäß Anspruch 1 bzw. von deren Säureadditions-Salzen und Metallsalz-Komplexen zur Bekämpfung von Pilzen bzw. zur Bekämpfung von Parasiten.

6. Verfahren zur Herstellung von fungiziden und parasitiziden Mitteln, dadurch gekennzeichnet, daß man mercapto-substituierte Hydroxyethyl-(triazol-1-yl)-Derivate der Formel (I) gemäß Anspruch 1 bzw. deren Säureadditionssalze oder Metallsalz-Komplexe mit Streckmitteln und/oder oberflächenaktiven Stoffen vermischt.

7. Verfahren zur Bekämpfung von Pilzen bzw. von Parasiten, dadurch gekennzeichnet, daß man mercapto-substituierte Hydroxyethyl-(triazol-1-yl)-Derivate der Formel (I) gemäß Anspruch 1 oder deren Säure-additions-Salze oder Metallsalz-Komplexe auf die Pilze oder Parasiten und/oder deren Lebensraum ausbringt.

8. Oxirane der Formel

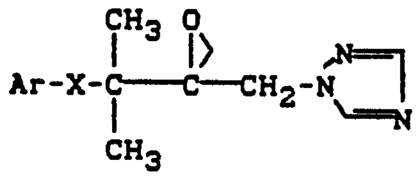

(II)

in welcher

Ar für gegebenenfalls substituiertes Aryl steht, und

X für Sauerstoff, Schwefel, eine direkte Bindung sowie für die Gruppierungen $-CH_2-$, $-CH_2-CH_2-$, $-O-CH_2-$, $-SCH_2-$, $-O-CH_2-CH_2-$und $-S-CH_2-CH_2-$steht, wobei das Heteroatom mit dem Aryl-Rest verbunden ist, wenn X für $-OCH_2-$, $-SCH_2-$, $-O-CH_2-CH_2-$oder $-S-CH_2-CH_2-$steht.

9. Verfahren zur Herstellung von Oxiranen der Formel

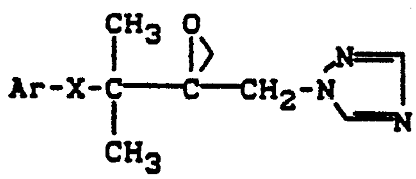

(II)

in welcher

Ar für gegebenenfalls substituiertes Aryl steht, und

X für Sauerstoff, Schwefel, eine direkte Bindung sowie für die Gruppierungen $-CH_2-$, $-CH_2-CH_2-$, $-O-CH_2-$, $-SCH_2-$, $-O-CH_2-CH_2-$und $-S-CH_2-CH_2-$steht, wobei das Heteroatom mit dem Aryl-Rest verbunden ist, wenn X für $-OCH_2-$, $-SCH_2-$, $-O-CH_2-CH_2-$oder $-S-CH_2-CH_2-$steht,

dadurch gekennzeichnet, daß man Ketone der Formel

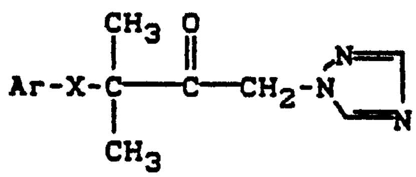

(IV)

in welcher

Ar und X die oben angegebene Bedeutung haben, entweder

α) mit Dimethyloxosulfonium-methylid der Formel

$$\overset{\delta^+\delta^-}{(CH_3)_2SOCH_2} \qquad (V)$$

in Gegenwart eines Verdünnungsmittels umsetzt,

oder

β) mit Trimethylsulfonium-methylsulfat der Formel

$[(CH_3)_3S^{\oplus}]\ CH_3SO_4^{\ominus}$ (VI)

in Gegenwart eines Verdünnungsmittels und in Gegenwart einer Base umsetzt.